# EUROPÄISCHE PATENTSCHRIFT

(11) **EP 0 764 655 B1**
(45) Veröffentlichungstag und Bekanntmachung des Hinweises auf die Patenterteilung: **14.03.2001**
(21) Anmeldenummer: 96113234.7
(22) Anmeldetag: 19.08.1996
(51) Int. Cl.: C07H 3/06, C07H 5/04, A61K 7/00

(54) **Kationische, ungesättigte Saccharide sowie daraus hergestellte Polymerisate und deren Verwendung**
Cationic unsaturated saccharides, polymers produced from them and their use
Saccharides insaturés cationiques, polymères produits par ces saccharides et leur utilisation

(30) Priorität: 25.08.1995 DE 19531264
(43) Veröffentlichungstag der Anmeldung: 26.03.1997
(73) Patentinhaber: Clariant GmbH, 65929 Frankfurt am Main (DE)
(72) Erfinder: Dönges, Reinhard, Dr., 65812 Bad Soden (DE); Ehrler, Rudolf, Dr., 65439 Flörsheim (DE)

(56) Entgegenhaltungen:
- EP-A- 0 166 089
- DE-A- 2 952 507
- MAKROMOLEKULARE CHEMIE, MACROMOLECULAR CHEMISTRY AND PHYSICS, Bd. 191, 1990, BASEL CH, Seiten 517-528, XP000611640 J.KLEIN ET AL.: "Poly(vinylsaccharide)s, 7."
- MAKROMOLEKULARE CHEMIE, MACROMOLECULAR CHEMISTRY AND PHYSICS, Bd. 188, 1987, BASEL CH, Seiten 1217-1232, XP000611641 J.KLEIN ET AL.: "Poly(vinylsaccharide)s, 2."

## Beschreibung

Kationische, ungesättigte Saccharide sowie daraus hergestellte Polymerisate und deren Verwendung

Kationische Polymere haben ein großes Anwendungsspektrum. Es reicht von kosmetischen Formulierungen, insbesondere für die Haarpflege, über lonenaustauscher, bis hin zu Papierhilfsmitteln und zur Abwasserbehandlung.

Von besonderem Interesse für Anwendungen in biologischen Systemen und für medizinische Applikationen, zum Beispiel als Depot-Speicher für Medikamente, sind besonders solche Polymere, deren Monomerbausteine auf Kohlenhydraten basieren. Nichtionische Monomerbausteine auf Kohlenhydrat-Basis sind bereits mehrfach in der Literatur beschrieben. So lassen sich Deoxy-aminozucker mit reaktiven Vinylkomponenten auf Basis von Acrylaten in die entsprechenden Acrylamide überführen, die ihrerseits polymerisierbar sind (Makromol. Chem. 191, 517-528 (1990) und Makromol. Chem. 188, 1217 (1987)). Als weitere polymerisierbaren Kohlenhydratmonomeren sind die Vinyl-β-D-glycoside (Chem. Ber. 85, 175 (1952), peracylierte 1-0-Acryloylzucker (DE 2 952 507), N-p-Vinylbenzyl-gluconamide (Polym. J. 17, 567 (1985) und Glycosylethylmethacrylate (J. Appl. Polymer Science 52, 1759-1763 (1994)) bekannt.

Nur sehr wenig ist über Monomere auf der Basis von Kohlenhydraten bekannt, die kationischer Natur sind. In EP 0 166 089 werden polymerisierbare Monomere beschrieben, die so aufgebaut sind, daß die reaktive, polymerisierbare Einheit über eine Glycosid-Bindung mit einem Kohlenhydrat verknüpft ist. Diese Verbindungen haben einige Nachteile. Zum einen geht ihre Synthese über mehrere Stufen. Bei der Synthese der Vorstufe, der Glycosidierung mit 3-Chlor-1,2-propandiol, entstehen oligomere Glycoside, überschüssiges Chlorpropandiol muß destillativ abgetrennt werden. Aufgrund der glycosidischen Bindung ist eine Stabilität der Polymeren im sauren pH-Bereich nicht gegeben.

Aufgabe der Erfindung war es daher, neue kationische, polymerisierbare Monomere auf Kohlenhydrat-Basis zur Verfügung zu stellen, die leicht in hoher Ausbeute und Reinheit herzustellen sind und sowohl im sauren als auch im alkalischen pH-Bereich stabil sind.

Gegenstand der Erfindung sind kationische, ungesättigte Saccharide der Formel I

R⁵R⁴C = CHR³-CH₂-N^{⊕}R¹R²-CH₂-CHR³ =CR⁴R⁵ X^{⊖} (I)

worin
- R¹: einen Deoxysaccharidrest mit 1 bis 20 Monomereinheiten,
- R²: die gleiche Bedeutung wie R¹ hat oder C₁-C₂₀-Alkyl, vorzugsweise C₁-C₄-Alkyl, C₂-C₂₀-Alkenyl, vorzugsweise C₂-C₄-Alkenyl, C₁-C₄-Hydroxyalkyl, C₁-C₄-Alkylphenyl oder eine Polyoxyethylen-, Polyoxypropylen- oder Polyethylenimin-Gruppe mit jeweils 1 bis 10 Ethylenoxid-, Propylenoxid- oder Ethylenimin-Einheiten,
- R³, R⁴ und R⁵: Wasserstoff oder Methyl und
- X: ein Halogen-, Tosylat- oder Allylsulfat-Ion bedeuten.

Diese kationischen, ungesättigten Saccharide werden hergestellt durch Umsetzung von Deoxyaminosacchariden der Formel II

R¹R²NH (II)

mit Allylverbindungen der Formel III

XCH-CHR³ = CR⁴R⁵ (III)

wobei R¹, R², R³, R⁴, R⁵ und X die oben genannten Bedeutungen haben.

Bevorzugt sind Verbindungen der Formel I worin R₁ ein 1-Deoxyglucityl, 1-Deoxygalactityl, 1-Deoxylactityl oder 1-Deoxymaltityl-rest ist.

Die Deoxyaminosaccharide der Formel II sind an sich bekannt (US 2,830,983; EP-A-0,255,033; Makromol. Chem. 191, 517-528 (1990). Sie werden hergestellt durch reduktive Aminierung von Sacchariden. Als Saccharide können alle Mono-, Di- und Oligosaccharide, die eine Aldehyd- oder Ketofunktion enthalten (sogenannte reduzierende Zucker) eingesetzt werden mit 1 bis 20, vorzugsweise 1, Saccharideinheiten, wie z.B. Glucose, Maltose, Fructose, Isomaltulose, Galactose, Lactose und Dextrine.

Der Begriff Saccharide umfaßt auch solche Kohlenhydrate, deren Hydroxylgruppen ganz oder teilweise in bekannter Weise durch Schutzgruppen blockiert sind, beispielsweise durch Acylierung (Acetylgruppen), Ketalisierung (Isopropyliden-, Benzylidengruppen), Veretherung (Methyl-, Hydroxyalkyl-, Carboxymethylgruppen).

Bevorzugt sind N-Alkyl-deoxyamino-monosaccharide, d.h. solche Verbindungen der Formel II, worin R¹ ein Deoxymono-Saccharid und R² C₁-C₄-Alkyl bedeutet. So erhält man beispielsweise aus Methylamin und Glucose das 1-(N-Methylamino)-1-deoxysorbit (N-Methylglucamin), aus Propylamin und Maltose das 1-(N-Propylamino)-1-deoxymaltit (N-Methylglucamin), aus Propylamin und Maltose das 1-(N-Propylamino)-1-deoxymaltit (N-Methylmaltamin) oder aus Methoxypropylamin und Isomaltulose ein Gemisch aus 2-(Methoxypropylamino)-2-deoxy-D-glucopyranosylmannit und -sorbit.

Durch Umsetzung dieser Deoxyaminosaccharide mit 2,0 bis 2,5 Moläquivalent einer Allylverbindung der Formel III in Gegenwart von einem Moläquivalent einer Base wie zum Beispiel Alkalihydroxid, vorzugsweise NaOH, erhält man die Verbindungen der Formel I in quantitativer Ausbeute. Als Allylverbindung der Formel III kommt von allem Allylchlorid in Frage, daneben aber auch Diallylsulfat und Allyltosylat.

Als Lösemittel für die Umsetzung eignen sich Wasser, wasserlösliche Alkohole wie Methanol, Ethanol, Isopropanol oder Ketone wie Aceton, Methylethylketon sowie aprotisch dipolare Lösemittel wie Dimethylsulfoxid, Dimethylformamid, N-Methylpyrrolidon oder Mischungen aus diesen Lösemitteln. Bevorzugtes Lösemittel ist Wasser.

Zweckmäßigerweise geht man so vor, daß man eine 20 bis 70 %ige, vorzugsweise 30 bis 60 %ige Lösung des Deoxyaminosaccharids II in dem gewünschten Lösungsmittel vorlegt und dann bei 40 bis 100°C, vorzugsweise jedoch am Siedepunkt der Allylverbindung, die Allylverbindung und das Alkalihydroxid so zutropft, daß ein leicht alkalischer pH-Wert von 8 bis 10, vorzugsweise jedoch 8,5 bis 9,5 vorliegt. Die Zutropfdauer hängt von der Reaktionstemperatur ab. Werte von 1 h bis 5 h sind üblich. In einer Phase der Nachreaktion wird solange unter pH-Kontrolle weitergerührt, bis die gesamte Allylverbindung abreagiert ist. Typische Zeiten für die Dauer der Nachreaktion sind 1 h bis 10 h, vorzugsweise 5 bis 8 h. Danach wird abgekühlt und mit einer Mineralsäure, vorzugsweise Salzsäure, auf pH 6,5 bis 7,5 gestellt. Die so erhaltene Lösung enthält nun die Verbindungen der Formel I in gebrauchsfertiger Form. Die Lösung enthält jedoch noch in molarer Menge ein Salz, das bei der Neutralisation während der Reaktion entstanden ist, sowie kleinere Mengen des zu der Allylverbindung III korrespondierenden Allylalkohols. Sollen diese Produkte abgetrennt werden, so dampft man die Reaktionslösung so weit wie möglich im Vakuum ein, wobei niedrigsiedende und wasserdampfflüchtige Verunreinigungen entfernt werden. Den Rückstand versetzt man mit einem Lösemittel, welches, gegebenenfalls durch Erwärmen, die Ammoniumverbindung löst, das abzutrennende Salz jedoch als Bodensatz zurückläßt. Als geeignete Lösemittel haben sich Propanole und Ethanol, sowie Mischungen daraus erwiesen. Die Lösung wird nun filtriert und das Filtrat erneut eingedampft. So erhält man die erfindungsgemäßen Produkte in analytisch und spektroskopisch reiner Form, meist als hygroskopische, sehr viskose Flüssigkeiten. Diese lassen sich mit Wasser zu gut handhabbaren Lösungen verdünnen. Bevorzugt sind Konzentrationen von 40 bis 70 Gew.-%. Als Beweis für die Vollständigkeit der Reaktion kann der Basenstickstoffwert des Produkts, bestimmt durch Titration mit Perchlorsäure-Maßlösung in Eisessig, herangezogen werden. Er ist kleiner als 0,1 %. Im ¹H-NMR-Spektrum absorbieren die Vinyl-H-atome zwischen 5,5 und 6,2 ppm, das ¹³C-NMR-Spektrum weist die Absorptionen von exakt 2 Vinyl-C-Atomen im Bereich um 130 ppm auf.

Die so erhaltenen erfindungsgemäßen Produkte können entweder als Lösungen oder in Substanz durch Homopolymerisation, Copolymerisation oder Pfropfcopolymerisation in hochmolekulare Produkte überführt werden. Die Monomeren der Formel I ergeben bei dieser Polymerisation wiederkehrende Einheiten der Formel

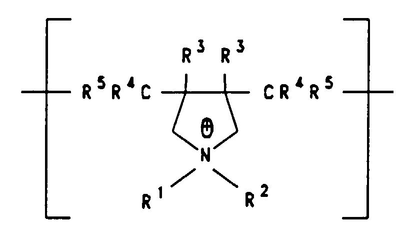

Als Comonomere eignen sich alle polymerisierbaren Vinylmonomeren, beispielsweise die Derivate der Acrylsäure, Styrole, Vinylether, -sulfonate, -phosphonate, -acetate und acetamide oder weitere Diallylammoniumverbindungen. Besonders geeignet für die Lösungscopolymerisation sind Monomere, die wasserlöslich sind. Die Pfropfcopolymerisation kann auf alle polymeren Verbindungen erfolgen, die in der Lage sind, Radikale zu bilden. Besonders geeignet als Pfropfgrundlage sind jedoch polymere Kohlenhydrate und deren Derivate.

Die Arbeitsweisen zur Herstellung derartiger hochmolekularer Verbindungen sind aus dem Stand der Technik zur Genüge bekannt, beispielsweise die Polymerisation in Substanz, Lösungspolymerisation, Suspensionspolymerisation und Emulsionspolymerisation. Als Radikalinitiatoren können beispielsweise Peroxide wie Wasserstoffperoxid, Di-tert. Butylperoxid, Dibenzoylperoxid, Dilauroylperoxid, Azoverbindungen wie 2,2'-Azobis-(isobutylnitril), Azobis(2-amidinopropan)dihydrochlorid, oder Alkali- und Ammoniumpersulfate, Redoxinitiatorsysteme, UV-, Beta- oder Gammastrahlung dienen.

Zu bevorzugen ist eine Lösungspolymerisation in Wasser, wobei als Radikalinitiatoren zweckmäßigerweise wasserlösliche Produkte wie Alkali- oder Ammoniumpersulfate oder quartäre Azoamidin-Salze wie zum Beispiel Azobis(2-amidinopropan)dihydrochlorid eingesetzt wird. Die Aufarbeitung der Polymeren richtet sich nach der Art der vorliegenden Polymeren. So können Homopolymerisate durch Fällung aus einem geeigneten Lösungsmittel isoliert werden, sie können aber auch direkt als fertige Lösungen, zum Beispiel als Flockungsmittel eingesetzt werden. Copolymerisate und Pfropfcopolymerisate werden durch Umfällung aus einem geeigneten Lösungsmittel oder Lösungsmittelgemisch gereinigt. Als Fällungsmittel haben sich Aceton, Isopropanol, Ethanol und Methanol sowie deren Mischungen mit Wasser als geeignet erwiesen. Die Charakterisierung der Copolymeren erfolgt elementaranalytisch durch Verbrennungsanalyse und durch potentiometrische Chlorid-Titration.

Die so erhaltenen Homo-, Co- oder Pfropfpolymerisate werden benutzt als Flockungsmittel für saure Abwässer. Wegen ihrer besonderen Hautfreundlichkeit eignen sie sich darüberhinaus auch als Verdickungsmittel in Kosmetika.

### Beispiele

### 1. Herstellung von Diallylmethylglucammoniumchlorid

In einem 3 l Mehrhalskolben mit Rückflußkühler, Rührer, 2 Tropfrichter, pH-Elektrode und Stickstoffeinlaß werden 1340 g einer 41 %igen wäßrigen Methylglucamin-Lösung (entspr. 2,9 mol Methylglucamin) vorgelegt. In den ersten Tropfrichter werden 475 g (6,2 mol) 3-Chlor-1-propen (Allylchlorid), in den anderen eine Lösung von 116 g (2,9 mol) Natriumhydroxid in 200 g Wasser vorgelegt. Zu Beginn der Reaktion beträgt der pH-Wert der Lösung 12,0. Man erwärmt den Ansatz auf 50°C und läßt 3-Chlor-1-propen zutropfen. Nach 2 Stunden ist etwa die Hälfte zugetropft und der pH-Wert auf 9 gefallen. Das restliche 3-Chlor-1-propen wird nun weiter innerhalb von 3 Stunden zugetropft, wobei gleichzeitig durch Zugabe der Natronlauge aus dem zweiten Tropfrichter ein pH-Wert von 9 bis 9,5 gehalten wird. Es wird noch 8 h bei einem pH-Wert von 9,5 und einer Temperatur von 50°C weitergerührt. Der Ansatz wird auf Raumtemperatur abgekühlt und mit ca. 9 g 37 %iger Salzsäure auf einen pH-Wert von 7 gebracht. Zur Aufarbeitung wird die Lösung am Rotationsverdampfer eingeengt, wobei zur azeotropen Entfernung des Wassers mehrfach Isopropanol zugesetzt wird. Der Rückstand wird in 2 l ca. 60°C warmem Isopropanol gelöst und vom Rückstand (Natriumchlorid) abfiltriert. Der Rückstand wird noch zweimal mit warmem Isopropanol nachgewaschen, getrocknet und ausgewogen (156 g Natriumchlorid). Die gesammelten Isopropanol-Lösungen werden am Rotationsverdampfer bis zur Trockene eingedampft. Restliches Isopropanol wird mit Wasser azeotrop ausgeschleppt. Das Produkt fällt als farbloses, hochviskoses Harz an.

Der Chloridgehalt (potentiometrisch) beträgt 11,1 % (Theorie 11,4 %), der Restbasenstickstoff 0,04 %. Das Produkt wird mit 1500 g Wasser zu einer leicht viskosen 63 %igen Lösung verdünnt. Im ¹H-NMR-Spektrum (300 MHz in DMSO-d₆) sind neben den Protonen der Methylgruppe und des Kohlenhydratteils noch die abgesetzten Signale der endständigen Vinylprotonen bei 5,50 bis 5,75 ppm und des innenstehenden Vinylprotons bei 6,00 bis 6,20 ppm zu erkennen, jeweils mit typischem Aufspaltungsmuster und einem Intensitätsverhältnis Vinyl/Methyl von 2:1. Im ¹³C-NMR-Spektrum (75,4 MHz in DMSO-d₆) sind 10 verschiedene Signale für Kohlenstoffatome zu erkennen: 48,4 ppm, 63,7 ppm, 63,8 ppm, 64,6 ppm, 67,3 ppm, 70,4 ppm, 71,0 ppm, 71,9 ppm, 126,1 ppm und 128,6 ppm. Neben diesen Signalen sind außer dem Lösungsmittel keine weiteren Signale zu erkennen.

### 2. Herstellung von Diallylmethyllactammoniumchlorid

In einem 500 ml Mehrhalskolben mit Rückflußkühler, KPG-Rührung, 2 Tropfrichter, pH-Elektrode und Stickstoffeinlaß werden 181 g einer 36 %igen wäßrigen Methyllactamin-Lösung (hergestellt durch reduktive Aminierung von Lactose mit Methylamin) vorgelegt. In den einen Tropfrichter werden 40,5 g 3-Chlor-1-propen (Allylchlorid), in den anderen eine Lösung von 10 g Natriumhydroxid in 25 g Wasser vorgelegt. zu Beginn der Reaktion beträgt der pH-Wert der Lösung 11,8. Man erwärmt den Ansatz auf 50°C und läßt 3-Chlor-1-propen zutropfen. Nach 2 Stunden ist etwa die Hälfte zugetropft und der pH-Wert auf 9 gefallen. Das restliche 3-Chlor-1-propen wird nun weiter innerhalb von 3 Stunden zugetropft, wobei gleichzeitig mittels der Natronlauge-Lösung ein pH-Wert von 9 bis 9,5 gehalten wird. Es wird noch 8 h bei einem pH-Wert von 9,5 und einer Temperatur von 50°C weitergerührt. Der Ansatz wird auf Raumtemperatur abgekühlt und mit 37 %iger Salzsäure auf einen pH-Wert von 7 gebracht. Zur Aufarbeitung wird die Lösung am Rotationsverdampfer eingeengt, wobei zur azeotropen Entfernung des Wassers mehrfach Isopropanol zugesetzt wird. Der Rückstand wird in 1 l siedendem Ethanol gelöst und filtriert Nach Trocknung verbleiben 14,3 g Natriumchlorid. Die ethanolische Lösung wird am Rotationsverdampfer bis zur Trockene eingedampft. Restliches Ethanol wird mit Wasser azeotrop ausgeschleppt. Das Produkt fällt als farbloses, hochviskoses Harz an. Das Produkt hat noch 0,06 % Basenstickstoff. Es wird mit 160 g Wasser zu einer leicht viskosen, 52 %igen Lösung verdünnt.

### 3. Homopolymerisation von Diallylmethylglucammoniumchlorid

170 g der 63 %igen Lösung von Diallylmethylglucammoniumchlorid aus Beispiel 1 werden in einem Dreihalskolben mit Stickstoffeinlaß und Septum vorgelegt. Es wird mehrmals evakuiert und mit Stickstoff belüftet. Es wird unter Inertatmosphäre auf 80°C aufgeheizt und eine Lösung von 0,80 g Ammoniumpersulfat in 5 ml entgastem Wasser über das Septum zugespritzt. nach 24, 48 und 72 Stunden werden noch einmal jeweils 0,80 g Ammoniumpersulfat in 5 ml Wasser auf diese Weise zugegeben. Man erhält eine hochviskose Polymerlösung.

### 4. Copolymerisation von Acrylamid und Methyldiallylglucammoniumchlorid

In einer Apparatur wie in Beispiel 3 beschrieben werden in 150 g entgastem Wasser 56,6 g Acrylamid und 99,3 g Methyldiallylglucammoniumchlorid-Lösung (63 Gew.-%) aus Beispiel 1 vorgelegt. Es wird auf 60°C aufgeheizt und über das Septum eine Lösung von 1,0 g 2,2'-Azobis(2-amidinopropan)dihydrochlorid in 3 ml Wasser langsam über 30 min zugegeben. Man rührt 5 Stunden bei 80°C nach und erhält eine klare, viskose Lösung. Diese wird unter starker Rührung (Ultra Turrax) in 2 l Methanol gegossen, wobei das Polymer ausfällt. Dieses wird noch zweimal mit 1 l Methanol aufgerührt und abgesaugt. Das Produkt wird bei 70°C getrocknet. Man erhält 92 g farbloses Pulver mit einem Chlorid-Gehalt von 4,01 % und einer Viskosität von 333 mPas, 10 Gew.-%ig in Wasser, gemessen mit einem Höppler-Viskosimeter.

### 5. Copolymerisation von Acrylamid und Methyldiallylglucammoniumchlorid

In einer Apparatur wie in Beispiel 3 beschrieben werden in 450 g entgastem Wasser 113,8 g Acrylamid und 99,3 g Methyldiallylglucammoniumchlorid-Lösung (63 Gew.-%) aus Beispiel 1 vorgelegt. Es wird auf 60°C aufgeheizt und über das Septum eine Lösung von 2,0 g 2,2'-Azobis(2-amidinopropan)dihydrochlorid in 6 ml Wasser langsam über 30 min zugegeben. Man rührt 5 Stunden bei 80°C nach und erhält eine klare, viskose Lösung. Diese wird unter starker Rührung (Ultra Turrax) in 2,5 l Methanol gegossen, wobei das Polymer ausfällt. Dieses wird noch zweimal mit 1 l Methanol aufgerührt und abgesaugt. Das Produkt wird bei 70°C getrocknet. Man erhält 150 g farbloses Pulver mit einem Chlorid-Gehalt von 2,40 % und einer Viskosität von 2210 mPas, 10 Gew.-%ig in Wasser, gemessen mit einem Höppler-Viskosimeter.

### 6. Copolymerisation von Acrylsäure und Methyldiallylglucammoniumchlorid

In einer Apparatur wie in Beispiel 3 beschrieben werden in 100 g entgastem Wasser 14,4 g Acrylsäure und 99,3 g Methyldiallylglucammoniumchlorid-Lösung (63 Gew.-%) aus Beispiel 1 vorgelegt. Es wird auf 60°C aufgeheizt und über das Septum eine Lösung von 0,5 g 2,2'-Azobis(2-amidinopropan)dihydrochlorid in 2 ml Wasser langsam über 2 Stunden zugegeben. Man rührt 2 Stunden bei 80°C nach und erhält eine klare, nur gering viskose Lösung. Diese wird mit 30 %iger Natronlauge auf einen pH-Wert von 10,5 eingestellt, mit 500 ml Isopropanol versetzt und im Vakuum eingedampft. Der Rückstand wird unter starker Rührung (Ultra Turrax) in 2,5 l Methanol gegossen, wobei das Polymer ausfällt. Dieses wird noch zweimal mit 2 l Methanol aufgerührt und abgesaugt. Das Produkt wird bei 70°C getrocknet. Man erhält 41,1 g farbloses Pulver mit einem Chlorid-Gehalt <0,1 %, einem Stickstoffgehalt von 2,7 % und einer Viskosität von 970 mPas, 10 Gew.-%ig in Wasser, gemessen mit einem Höppler-Viskosimeter.

### 7. Copolymerisation von 2-Acrylamido-2-methylpropansulfonsäure und Methyldiallylglucammoniumchlorid

In einer Apparatur wie in Beispiel 3 beschrieben werden in 100 g entgastem Wasser 82 g 2-Acrylamido-2-methylpropansulfonsäure und 99,3 g Methyldiallylglucammoniumchlorid-Lösung (63 Gew.-%) aus Beispiel 1 vorgelegt. Es wird auf 60°C aufgeheizt und über das Septum eine Lösung von 0,5 g 2,2'-Azobis(2-amidinopropan)dihydrochlorid in 2 ml Wasser in einem Zeitraum von 15 min zugegeben. Man rührt 5 Stunden bei 80°C nach und erhält eine klare viskose Lösung. Diese wird mit 30 %iger Natronlauge auf einen pH-Wert von 7 eingestellt, mit 500 ml Isopropanol versetzt und im Vakuum eingedampft. Der Rückstand wird unter starker Rührung (Ultra Turrax) in 2,5 l Ethanol gegossen, wobei das Polymer ausfällt. Dieses wird noch zweimal mit 2 l Ethanol aufgerührt und abgesaugt. Das Produkt wird bei 70°C getrocknet. Man erhält 108 g farbloses Pulver mit einem Chlorid-Gehalt von 0,4 %, einem Stickstoffgehalt von 5,5 % und einem Schwefelgehalt von 9,4 %.

### 8. Pfropfcopolymerisation von Methyldiallylglucammoniumchlorid auf Carboxymethylcellulose

In einem 2 l Rührreaktor mit Rückflußkühler, Stickstoffüberleitung und Innenthermometer werden bei Raumtemperatur 96,8 g Carboxymethylcellulose CB 30000 (Tylose®, Viskosität ca. 30000 mPas 2 %ig in Wasser, DS = 0,68), 350 g Dimethoxyethan und 104 g Diallylmethylglucammoniumchlorid Monomerlösung (63 %ig, aus Beispiel 1) vorgelegt. Der Ansatz wird auf 50°C aufgeheizt und unter Durchleiten eines leichten Stickstoffstromes und langsamem Rühren entgast. Dann gibt man 2,0 g 2,2'-Azobis(2-amidinopropan)dihydrochlorid, fein gepulvert, zu und rührt 1 h bei Raumtemperatur. Der Ansatz wird auf 70°C aufgeheizt und 16 h bei 70°C langsam gerührt. Das Produkt wird abgesaugt und 6 mal mit 60 %igem wäßrigen Isopropanol und zweimal mit Aceton ausgewaschen und bei 70°C getrocknet. Man erhält 101 g eines farblosen Pulvers mit einem Natriumgehalt von 5,22 %, entsprechend einem Substitutionsgrad bezüglich Carboxylat von 0,68 Mol/Anhydroglucoseeinheit, einem Stickstoffgehalt von 0,8 %, entsprechend einem mittleren Pfropfgrad von 0,17 Mol/Anhydroglucoseeinheit. Die Viskosität einer 2 %igen Lösung des gepfropften Polymers beträgt 3490 mPas, gemessen an einem Höppler-Viskosimeter.

### 9. Pfropfcopolymerisation von Methyldiallylglucoammoniumchlorid auf Hydroxyethylcellulose

In einem 2 l Rührautoklav werden bei Raumtemperatur 100 g Hydroxyethylcellulose H10000 (Tyose®, Viskosität ca. 10000 mPas 2 %ig in Wasser, MS = 2,40), 450 g Cyclohexan und 85 g Diallylmethylglucammoniumchlorid Monomerlösung (63 %ig, aus Beispiel 1) und 30 g Dibenzoylperoxid vorgelegt. Der Ansatz wird inertisiert und dann unter Rührung 6 h auf 90°C erhitzt. Nach Abkühlen wird das Produkt abgesaugt, 6 mal mit 80 %igem wäßrigen Isopropanol und zweimal mit Aceton ausgewaschen und bei 70°C getrocknet. Man erhält 84 g eines farblosen Pulvers mit einem Stickstoffgehalt von 0,6 %, entsprechend einem mittleren Pfropfgrad von 0,13 Mol/Anhydroglucoseeinheit. Die Viskosität einer 2 %igen Lösung des gepfropften Polymers beträgt 620 mPas, gemessen an einem Höppler-Viskosimeter.

## Patentansprüche

1. Kationische, ungesättigte Saccharide der Formel I
R⁵R⁴C = CHR³-CH₂-N^{⊕}R¹R²-CH₂-CHR³ = CR⁴R⁵ X^{⊖} (I)
worin
R¹ einen Deoxysaccharidrest mit 1 bis 20 Monomereinheiten,
R² die gleiche Bedeutung wie R¹ hat oder C₁-C₂₀-Alkyl, C₂-C₂₀-Alkenyl, C₁-C₄-Hydroxyalkyl, C₁-C₄-Alkylphenyl oder eine Polyoxyethylen-, Polyoxypropylen- oder Polyethylenimin-Gruppe mit jeweils 1 bis 10 Ethylenoxid-, Propylenoxid- oder Ethylenimin-Einheiten,
R³, R⁴ und R⁵ Wasserstoff oder Methyl und
X ein Halogen-, Tosylat- oder Allylsulfat-Ion
bedeuten.

2. Verbindungen der Formel I nach Anspruch 1, worin R¹ einen Deoxymonosaccharidrest, R² C₁-C₄-Alkyl oder C₁-C₄-Hydroxyalkyl, R³, R⁴ und R⁵ jeweils ein Wasserstoffatom und X ein Chlorid-Ion bedeuten.

3. Verbindungen der Formel I nach Anspruch 1, worin R¹ ein 1-Deoxyglucityl-, 1-Deoxygalactityl-, 1-Deoxylactityl- oder 1-Deoxymaltitylrest ist.

4. Verfahren zur Herstellung der Verbindungen der Formel I, nach Anspruch 1, dadurch gekennzeichnet, daß man Deoxyaminosaccharide der Formel II
R¹R²NH (II)
mit Allylverbindungen der Formel III
XCH-CHR³ = CR⁴R⁵ (III)
umsetzt, wobei R¹, R², R³, R⁴, R⁵ und X die in Anspruch 1 angegebenen Bedeutungen haben.

5. Homo-, Co- und Pfropfpolymerisate enthaltend ein Monomer der Formel I nach Anspruch 1.

6. Verwendung der Homo-, Co- und Pfropfpolymerisate nach Anspruch 5 als Flockungsmittel und als Verdickungsmittel für Kosmetika.

## Claims

1. A cationic unsaturated saccharide of the formula I
R⁵R⁴C = CHR³-CH₂-N^{⊕} R¹R²-CH₂-CHR³=CR⁴R⁵ X^{⊖} (I)
in which
R¹ is a deoxysaccharide radical having 1 to 20 monomer units,
R² has the same meaning as R¹ or is C₁-C₂₀-alkyl, C₂-C₂₀-alkenyl, C₁-C₄-hydroxyalkyl, C₁-C₄-alkylphenyl or a polyoxyethylene, polyoxypropylene or polyethyleneimine group having in each case 1 to 10 ethylene oxide, propylene oxide or ethyleneimine units,
R³, R⁴ and R⁵ are hydrogen or methyl and
X is a halogen, tosylate or allyl-sulfate ion.

2. A compound of the formula I as claimed in claim 1, in which R¹ is a deoxymonosaccharide radical, R² is C₁-C₄-alkyl or C₁-C₄-hydroxyalkyl, R³, R⁴ and R⁵ are each a hydrogen atom and X is a chloride ion.

3. A compound of the formula I as claimed in claim 1, in which R¹ is a 1-deoxyglucityl, 1-deoxygalactityl, 1-deoxylactityl or 1-deoxymaltityl radical.

4. A process for the preparation of a compound of the formula I as claimed in claim 1, which comprises reacting a deoxyaminosaccharide of the formula II
R¹R²NH (II)
with an allyl compound of the formula III
XCH-CHR³= CR⁴R⁵ (III)
in which R¹, R², R³, R⁴, R⁵ and X have the meanings given in claim 1.

5. A homo-, co- or graft polymer comprising a monomer of the formula I as claimed in claim 1.

6. The use of the homo-, co- or graft polymer as claimed in claim 5 as a flocculating agent and as a thickening agent for cosmetics.

## Revendications

1. Saccharides insaturés, cationiques, de formule I
R⁵R⁴C=CHR³-CH₂-N^{⊕}R¹R²-CH₂-CHR³=CR⁴R⁵ XΘ (I)
où
R¹ représente un reste déoxysaccharide possédant 1 à 20 motifs monomères,
R² possède la même signification que R¹ ou représente des groupes alkyle en C₁-C₂₀, alcényle en C₂-C₂₀, hydroxyalkyle en C₁-C₄, (alkyl en C₁-C₄)phényle ou un groupe polyoxyéthylène, polyoxypropylène ou polyéthylénimine présentant à chacun de 1 à 10 motifs oxyde d'éthylène, oxyde de propylène ou éthylénimine,
R³, R⁴ et R⁵ représentent un atome d'hydrogène ou un groupe méthyle et
X représente un ion halogène, un ion tosylate ou un ion alkylsulfate.

2. Composés de formule I selon la revendication 1, où R¹ représente un reste déoxymonosaccharide, R² représente un groupe alkyle en C₁-C₄ ou hydroxyalkyle en C₁-C₄, R³, R⁴ et R⁵ représentent chacun un atome d'hydrogène et X représente un ion chlorure.

3. Composés de formule I selon la revendication 1, où R¹ représente un reste déoxyglucityle, 1-déoxygalactityle, 1-déoxylactityle ou 1-déoxymaltityle.

4. Procédé de préparation des composés de formule I, selon la revendication 1, caractérisé en ce qu'on fait réagir des déoxymonosaccharides de formule II
R¹R²NH (II)
avec des composés d'allyle de formule III
XCH-CHR³=CR⁴R⁵ (III)
R¹, R², R³, R⁴, R⁵ et X possédant les significations données à la revendication 1.

5. Homopolymères, copolymères et copolymères greffés contenant un monomère de formule I selon la revendication 1.

6. Utilisation des homopolymères, copolymères et copolymères greffés selon la revendication 5 en tant que floculants et en tant qu'épaississants dans des produits cosmétiques.
